(19) Europäisches Patentamt
European Patent Office
Office européen des brevets

(11) Veröffentlichungsnummer: **0 298 032 A2**

(12) # EUROPÄISCHE PATENTANMELDUNG

(21) Anmeldenummer: **88810428.8**

(22) Anmeldetag: **22.06.88**

(51) Int. Cl.⁴: **C 07 C 109/10**
C 08 G 59/40, G 03 C 1/68

(30) Priorität: **01.07.87 CH 2484/87**

(43) Veröffentlichungstag der Anmeldung:
**04.01.89 Patentblatt 89/01**

(84) Benannte Vertragsstaaten:
**CH DE FR GB LI NL**

(71) Anmelder: **CIBA-GEIGY AG**
**Klybeckstrasse 141**
**CH-4002 Basel (CH)**

(72) Erfinder: **Duthaler, Rudolf, Dr.**
**Girenhaldenweg 17**
**CH-4126 Bettingen (CH)**

**Finter, Jürgen, Dr.**
**Zasiusstrasse 100**
**D-7800 Freiburg (DE)**

**Fischer, Walter**
**Vogesenstrasse 77**
**CH-4153 Reinach (CH)**

**Ramanathan, Visvanathan, Dr.**
**Fridensgasse 24**
**CH-4056 Basel (CH)**

(54) **Anthrachinoylcarbonsäurehydrazide, härtbare Zusammensetzungen und deren Verwendung.**

(57) Anthrachinone der Formel I

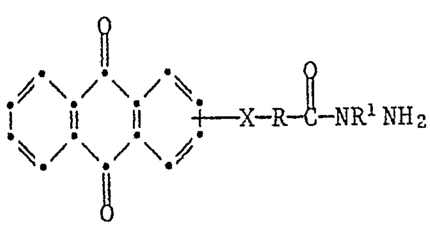

(I),

worin
X für die Gruppe $-CR^2R^3-$ steht, $R^2$ H, -CN oder $C_1$-$C_5$-Alkyl und $R^3$ H oder -CN sind, $R^1$ H oder $C_1$-$C_5$-Alkyl darstellt, und R eine direkte Bindung oder lineares oder verzweigtes $C_1$-$C_{18}$-Alkylen ist, das alleine oder zusammen mit der Gruppe $-CR^2R^3-$durch ein oder mehrere -O- unterbrochen sein kann, wenn $R^2$ und/oder $R^3$ nicht -CN sind, und härtbare Zusammensetzung, enthaltend a) ein Epoxidharz, b) gegebenenfalls einen Härter, c) ein Anthrachinon der Formel I und d) einen Aminoalkohol. Die gehärteten Zusammensetzungen sind lichtempfindlich und eignen sich zur Her stellung von Ueberzügen und metallischen Abbildungen durch stromlose Metallabscheidung.

EP 0 298 032 A2

## Beschreibung

### Anthrachinoylcarbonsäurehydrazide, härtbare Zusammensetzungen und deren Verwendung

Die Erfindung betrifft Anthrachinoylcarbonsäurehydrazide, eine härtbare Zusammensetzung aus a) einem Epoxidharz, b) gegebenenfalls einem Härter, c) einem Anthrachinoncarbonsäurehydrazid und d) einem Aminoalkohol, sowie deren Verwendung zur Herstellung von metallischen Ueberzügen oder Abbildungen.

In der EP-A-0 112 798 sind lichtempfindliche, vernetzte Reaktionsprodukte auf der Basis von Epoxidharzen beschrieben. Unter Mitverwendung von Metallsalzen der Gruppen Ib und VIII des Periodensystems der Elemente können durch Belichtung Metallkeime erzeugt werden, die man durch stromlose Metallabscheidung verstärken kann. Es ist wünschenswert, auf lichtempfindlichen Epoxidharzen direkt und ohne Mitverwendung von Metallsalzen metallische Ueberzüge oder Abbildungen durch stromlose Metallabscheidung zu erzeugen.

Ein Gegenstand der Erfindung sind Anthrachinone der Formel I

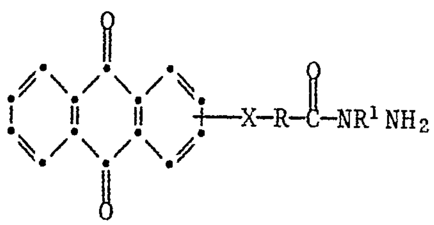 (I),

worin

X für die Gruppe $-CR^2R^3-$ steht, $R^2$ H, -CN oder $C_1$-$C_5$-Alkyl und $R^3$ H oder -CN sind, $R^1$ H oder $C_1$-$C_5$-Alkyl darstellt, und R eine direkte Bindung oder lineares oder verzweigtes $C_1$-$C_{18}$-Alkylen ist, das alleine oder zusammen mit der $-CR^2R^3-$Gruppe durch ein oder mehrere -O- unterbrochen sein kann, wenn $R^2$ und/oder $R^3$ nicht -CN sind.

Die Gruppe

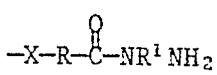

ist bevorzugt in 2-Stellung des Anthrachinons gebunden.

Eine bevorzugte Ausführungsform sind solche Anthrachinone der Formel I, worin X für die Gruppe $-CR^2-R^3-$ steht, in der $R^2$ H oder $C_1$-$C_5$-Alkyl und $R^3$ H bedeuten. Besonders bevorzugt sind dabei $R^2$ und $R^3$ je H.

Bei $R^1$ und $R^2$ kann es sich um lineares oder verzweigtes Alkyl handeln, z.B.ist als Alkyl bevorzugt Methyl oder Ethyl. $R^1$ steht bevorzugt für H oder Methyl. R kann als Alkylen verzweigt und bevorzugt linear sein. Es enthält bevorzugt 1 bis 12, besonders 1 bis 8 C-Atome.

In einer weiteren bevorzugten Ausführungsform stehen X für $-CH_2-$ und R für eine direkte Bindung oder lineares Alkylen mit 1 bis 8 C-Atomen.

R kann als Alkylen mit -O-, bevorzugt mit 1 bis 4, besonders 1 oder 2 -O-unterbrochen sein. In einer bevorzugten Ausführungsform entspricht das durch -O- unterbrochene Alkylen der Formel

$$-(C_mH_{2m}-O)_y-,$$

worin m eine Zahl von 2-4 vorzugsweise 2 oder 3 und besonders 2, und y eine Zahl von 1 bis 6, bevorzugt 1-4, besonders 1 oder 2 bedeuten.

Die Herstellung von Anthrachinon-2-carbonsäurehydrazid gelingt gemäss US-PS 2 884 424 nur durch Umsetzung eines entsprechenden Esters mit einem Ueberschuss Hydrazin zur Leucoform und dessen anschliessende Oxidation. Es wurde überraschend gefunden, dass die erfindungsgemässen Anthrachinone direkt bei der Umsetzung alkoholischer Lösungen der entsprechenden Ester mit Hydrazin gebildet werden.

Ein weiterer Gegenstand der Erfindung ist ein Verfahren zur Herstellung von Anthrachinonen der Formel I, das dadurch gekennzeichnet ist, dass man einen Anthrachinoncarbonsäureester der Formel II

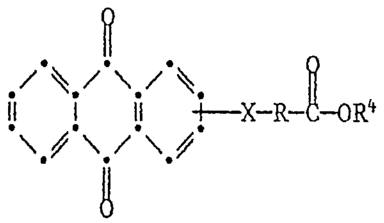 (II),

worin X und R die zuvor angegebenen Bedeutungen haben oder die Gruppe -XR- eine direkte Bindung darstellt und $R^4O$- für den Rest eines Alkohols steht, in Gegenwart eines Alkohols mit einem Hydrazin der Formel III

$HNR^1NH_2$ (III),

worin $R^1$ die zuvor angegebene Bedeutung hat, oder mit dessen Hydrat oder Salzen umsetzt.

$R^4$ in Formel II ist vorzugsweise $C_1$-$C_6$-, besonders $C_1$-$C_4$-Alkyl. Insbesondere steht $R^4$ für Methyl oder Ethyl.

Bei dem Alkohol handelt es sich bevorzugt um ein Alkanol, das insbesondere 1-4 C-Atome aufweist. Bevorzugt werden Alkanole eingesetzt, deren Alkylrest mit $R^4$ identisch ist. Besonders bevorzugt wird Ethanol verwendet.

Vorteilhaft wird die Reaktion unter Inertgasatmosphäre durchgeführt. Die Reaktionstemperatur kann 30 bis 180°C, vorzugsweise 50 bis 120°C betragen.

Das Alkanol kann als Lösungsmittel dienen. Es können zusätzlich andere inerte Lösungsmittel mitverwendet werden, z.B. Ether (Diethylether, Dioxan, Tetrahydrofuran), Cellosolve, Sulfoxide und Sulfone und N-alkylierte Säureamide.

Die Hydrazine der Formel III sind bekannt. Vorteilhaft wird Hydrazinhydrat verwendet. Salze können sich z.B. von Mineral- oder Carbonsäuren ableiten.

Die Ester der Formel II sind bekannt oder können nach an sich bekannten Verfahren hergestellt werden. Die Herstellung von (Anthrachino-2-yl)-essigsäureester durch Substitution von Anthrachinoylchlorid mit Malonsäurediestern ist in der JP-OS 55/89245 beschrieben. Analog können Malondinitril und Malonesternitrile verwendet werden. Die so erhält lichen Anthrachinoylmalonderivate können in bekannter Weise mit $C_1$-$C_5$-Alkylhalogeniden oder mit Carbalkoxyalkylhalogeniden umgesetzt werden. Die partielle oder teilweise Hydrolyse der Nitril- oder Estergruppen und gegebenenfalls Decarboxylierung ergibt nach der Veresterung weitere Ester der Formel II. Diese Umsetzungen können auch mit dem bekannten Anthrachinoylacetonitril durchgeführt werden.

Die Ester der Formel II können auch durch die bekannte Umsetzung von Anthracen mit Alkencarbonsäureestern und die anschliessende Oxidation z.B. mit Jones-Reagenz erhalten werden. Ferner ist ein stufenweiser Aufbau durch die Umsetzung von an sich bekannten Phenalkylcarbonsäureestern mit Phthalsäureanhydrid, anschliessende Cyclisierung zur Anthrachinonalkancarbonsäure und deren Veresterung möglich. Phenalkylcarbonsäureester sind auch durch die katalytische Hydrierung von Benzoylalkancarbonsäuren und einer gleichzeitigen oder anschliessenden Veresterung erhältlich.

Zur Herstellung von Estern der Formel II, worin R durch -O- unterbrochenes Alkylen ist, kann zuvor ein Halogenalkylanthrachinon mit einem Alkylendiol und danach gegebenenfalls Alkylenoxiden umgesetzt werden. Die erhaltenen Hydroxyverbindungen können dann mit Alkencarbonsäureestern oder Halogenalkylcarbonsäureestern umgesetzt werden.

Die Verbindungen der Formel I eignen sich zur Herstellung von lichtempfindlichen gehärteten Epoxidharzen. Ein weiterer Gegenstand der Erfindung ist eine härtbare Zusammensetzung, enthaltend

a) mindestens ein Epoxidharz mit durchschnittlich mehr als einer Epoxidgruppe im Molekül,

b) gegebenenfalls einen Härter für Epoxidharz,

c) mindestens ein Anthrachinon der Formel I, wobei die Gruppe -XR-zusätzlich eine direkte Bindung darstellt, und

d) mindestens ein primäres oder sekundäres aliphatisches Amin, das im aliphatischen Rest mindestens eine Hydroxylgruppe enthält.

Für die Verbindungen der Formel I gelten die zuvor angegebenen Bevorzugungen.

Das Epoxidharz enthält bevorzugt durchschnittlich mindestens 2 Epoxidgruppen im Molekül.

Als Epoxidharze kommen vor allem solche mit durchschnittlich mehr als einer an ein Heteroatom (z.B. Schwefel, vorzugsweise Sauerstoff oder Stickstoff) gebundenen Glycidylgruppe, β-Methylglycidylgruppe oder 2,3-Epoxycyclopentylgruppe in Frage; genannt seien insbesondere Bis-(2,3-epoxycyclopentyl)-ether, Di- bzw. Polyglycidylether von mehrwertigen aliphatischen Alkoholen, wie 1,4-Butandiol, oder Polyalkylenglykolen, wie Polypropylenglykole; Di- oder Polyglycidylether von cycloaliphatischen Polyolen, wie 2,2-Bis-(4-hydroxycyclohexyl)-propan; Di- bzw. Polyglycidylether von mehrwertigen Phenolen, wie Resorcin, Bis-(p-hydroxyphenyl)-methan, 2,2-Bis-(p-hydroxyphenyl)-propan (= Diomethan), 2,2-Bis-(4'-hydroxy-3',5'-dibromphenyl)-propan, 1,1,2,2-Tetrakis-(p-hydroxyphenyl)-ethan, oder von unter sauren Bedingungen erhaltenen Kondensationsprodukten von Phenolen mit Formaldehyd wie Phenol-Novolake und Kresol-Novolake; Di- bzw.

Poly-(β-methylglycidyl)-ether der oben angeführten mehrwertigen Alkohole oder mehrwertigen Phenole; Polyglycidylester von mehrwertigen Carbonsäuren, wie Phthalsäure, Terephthalsäure, $\Delta^4$-Tetrahydrophthalsäure und Hexahydrophthalsäure; N-Glycidylderivate von Aminen, Amiden und heterocyclischen Stickstoffbasen, wie N,N-Diglycidylanilin, N,N-Diglycidyltoluidin, N,N,O-Triglycidyl-p-aminophenol, N,N,N',N'-Tetraglycidyl-bis-(p-aminophenyl)-methan; Triglycidyl-isocyanurat; N,N'-Diglycidylethylenharnstoff; N,N'-Diglycidyl-5,5-dimethylhydantoin, N,N'-Diglycidyl-5-isopropyl-hydantoin, N,N-Methylen-bis-(N',N'-diglycidyl)-5,5-dimethylhydantoin, 1,3-Bis-(N-glycidyl-5,5-dimethylhydantoin)-2-glycidyloxypropan; N,N'-Diglycidyl-5,5-dimethyl-6-isopropyl-5,6-dihydro-uracil.

Eine bevorzugte Gruppe von Epoxidharzen sind glycidylierte Novolake, Hydantoine, Aminophenole, Bisphenole oder aromatische Diamine. Besonders bevorzugte Zusammensetzungen enthalten als Epoxidharz einen glycidylierten Kresolnovolak, Bisphenol-A-diglycidylether, mit Bisphenol-A vorverlängerten Bisphenol-A-diglycidylether, Hydantoin-N,N'-bisglycid, 1,3-Bis-(N-glycidyl-5,5-dimethylhydantoin)-2-glycidyloxypropan, p-Aminophenoltriglycid, Diaminodiphenylmethan-tetraglycid oder Mischungen hiervon.

Geeignet sind auch vorreagierte Addukte solcher Epoxide mit Härtern für Epoxide, z.B. das zuvor erwähnte Addukt aus Bisphenol-A-diglycidylether und Bisphenol-A.

Als Härter für Epoxidharze kommen saure oder basische Verbindungen in Frage. Als geeignete Härter seien z.B. genannt: Amine, wie aliphatische, cycloaliphatische oder aromatische, primäre, sekundäre und tertiäre Amine, z.B. Ethylendiamin, Hexamethylendiamin, Trimethylhexamethylendiamin, Diethylentriamin, Triethylentetramin, Tetraethylenpentamin, N,N-Dimethylpropylendiamin-1,3, N,N-Diethylpropylen-diamin-1,3,2,2-Bis-(4'-aminocyclohexyl)-propan, 3,5,5-Trimethyl-3-(aminomethyl)-cyclohexylamin ("Isophorondiamin"), Mannichbasen, wie 2,4,6-Tris-(dimethylaminomethyl)-phenol; m-Phenylendiamin, p-Phenylendiamin, Bis-(4-aminophenyl)-methan, Bis-(4-aminophenyl)-sulfon, m-Xylylendiamin; Addukte von Acrylnitril oder Monoepoxiden, wie z.B. Ethylenoxid oder Propylenoxid an Polyalkylenpolyamine, wie z.B. Diethylentriamin oder Triethylentetramin; Addukte aus Polyaminen, wie z.B. Diethylentriamin oder Triethylentetramin, im Ueberschuss und Polyepoxiden, wie z.B. Diomethanpolyglycidylethern; Addukte aus Monophenolen oder Polyphenolen und Polyamiden; Polyamide, insbesondere solche aus aliphatischen Polyaminen, wie z.B. Diethylentriamin oder Triethylentetramin, und di- oder trimerisierten ungesättigten Fettsäuren, wie z.B. dimerisierte Leinölfettsäure (VERSAMID®); Polysulfide (THIOKOL®); Anilin-Formaldehyde; mehrwertige Phenole, z.B. Resorcin, 2,2-Bis-(4-hydroxyphenyl)-propan oder Phenol-Formaldehyd-Harze; mehrbasische Carbonsäuren und ihre Anhydride, z.B. Phthalsäureanhydrid, $\Delta^4$-Tetrahydrophthalsäureanhydrid, Hexahydrophthalsäureanhydrid, 4-Methylhexahydrophthalsäureanhydrid, 3,6-Endomethylen-$\Delta^4$-tetrahydrophthalsäureanhydrid, 4-Methyl-3,6-endomethylen-$\Delta^4$-tetrahydrophthalsäureanhydrid (= Methylnadicanhydrid), 3,4,5,6,7-Hexachlor-3,6-endomethylen-$\Delta^4$-tetrahydrophthalsäureanhydrid, Bernsteinsäureanhydrid, Adipinsäureanhydrid, Trimethyladipinsäureanhydrid, Azelainsäureanhydrid, Sebacinsäureanhydrid, Maleinsäureanhydrid, Dodecylbernsteinsäureanhydrid; Pyromellitsäuredianhydrid, Trimellitsäureanhydrid, Benzophenontetracarbonsäuredianhydrid oder Gemische solcher Anhydride.

Eine bevorzugte Gruppe von Härtern sind Novolake, Polyamine und Carbonsäureanhydride.

Die erfindungsgemässe Zusammensetzung kann auch Härtungsbeschleuniger bzw. Polymerisationsinitiatoren oder thermische bzw. photochemische Härtungskatalysatoren enthalten. Beispiele sind: tertiäre Amine, deren Salze oder quaternäre Ammoniumverbindungen, z.B. 2,4,6-Tris-(dimethylaminomethyl)phenol, Benzyldimethylamin, 2-Ethyl-4-methylimidazol, Triamylammoniumphenolat; oder Alkalimetallalkoholate, wie z.B. Natriumhexantriolat; Mono- oder Polyphenole wie Phenol oder Diomethan oder Salicylsäure; Dicyandiamid; Bortrifluorid und seine Komplexe mit organischen Verbindungen, wie $BF_3$-Ether-Komplexe und $BF_3$-Amin-Komplexe, z.B. $BF_3$-Monoethylamin-Komplex, Acetoacetanilid-$BF_3$-Komplex; Phosphorsäure; Triphenylphosphit. Als photochemische Härtungskatalysatoren eignen sich Oniumsalze oder Metallkomplexesalze wie z.B. Diazoniumsalze aromatischer Amine, Triphenylsulfonium- oder Diphenyliodoniumsalze oder Cyclopentadienyleisenarensalze.

Härtungsbeschleuniger und -katalysatoren werden üblicherweise in einer Menge von 0,1-10 Gew.-% zugegeben, bezogen auf das Epoxidharz. Härter für Epoxidharze werden im allgemeinen in äquimolaren Menge verwendet, bezogen auf die Epoxidgruppen und funktionellen Gruppen eines Härters.

Die Zusammensetzung enthält das Anthrachinon der Formel I bevorzugt in einer Menge von 0,1-1, besonders 0,2-0,8 Mol/kg Epoxidharz, und das Hydroxylgruppen enthaltende Amin bevorzugt in einer Menge von 0,1-1,2, besonders 0,3-1 Mol/kg Epoxidharz. Zusätzliche Härter sind bevorzugt in einer Menge von 0,1-0,5, besonders 0,1-0,3 Mol/kg Epoxidharz enthalten.

Bei den Aminen der Komponente d) kann es sich um Hydroxylgruppen enthaltende aliphatische Amine mit 2 bis 30, vorzugsweise 2 bis 20 C-Atomen und mit 1 bis 3, vorzugsweise 1 Hydroxylgruppen im aliphatischen Rest handeln. Der aliphatische Rest kann linear oder verzweigt sein und durch -O- oder Aminogruppen unterbrochen sein. Bevorzugt enthält der aliphatische Rest primäre OH-Gruppen. In einer bevorzugten Ausführungsform entspricht das Amin der Formel IV

$$H-N(R^5)-C_yH_{2y+1-x}(OH)_x \quad (IV),$$

worin $R^5$ H, lineares oder verzweigtes $C_1$-$C_{18}$-Alkyl, $C_3$-$C_7$-Cycloalkyl, $C_6$-$C_{10}$-Aryl, $C_7$-$C_{18}$-Alkaryl, $C_7$-$C_{12}$-Aralkyl, $C_8$-$C_{18}$-Alkaralkyl oder die Gruppe $-C_yH_{2y+1-x}OH_x$ bedeutet, x für eine Zahl von 1 bis 3 und y für eine Zahl von 2 bis 12 stehen, wobei die Gruppe $C_yH_{2y}$ durch ein oder mehrere -O- oder $-NR^5-$ unterbrochen sein kann.

$R^5$ enthält als Alkyl bevorzugt 1 bis 12 und besonders 1 bis 6 C-Atome. $R^5$ ist als Alkyl bevorzugt Methyl oder Ethyl. Insbesondere stellt $R^5$ H dar. Als Cycloalkyl enthält $R^5$ vorzugsweise 5 oder 6 Ring-C-Atome und ist z.B. Cyclopentyl oder Cyclohexyl.

$R^5$ kann in der Bedeutung von Aryl z.B. Naphthyl und besonders Phenyl sein. $R^5$ in der Bedeutung von Alkaryl ist besonders $C_7$-$C_{18}$-Alkylphenyl, zum Beispiel Methyl-, Ethyl-, Dimethyl-, n- und i-Propyl-, n-, i- und t-Butyl-, Pentyl-, Hexyl-, Octyl-, Nonyl-, Decyl- und Dodecylphenyl. Bei $R^5$ in der Bedeutung von Aralkyl kann es sich um 1-oder 2-Phenyleth-1-yl oder besonders Benzyl handeln. Wenn $R^5$ Alkaralkyl ist, handelt es sich bevorzugt um Alkylbenzyl mit insbesondere 8 bis 14 C-Atomen, z.B. Methyl-, Ethyl-, Dimethyl-, n-und i-Propyl-, n-, i- und t-Butyl-, Pentyl-und Hexylbenzyl. Sofern die Gruppe $C_yH_{2y}$ durch -O- unterbrochen ist, kann es sich um Oxaalkylen Reste handeln, die z.B. der Formel $-R^6-(OR^7)_t-$ entsprechen können, worin $R^6$ und $R^7$ unabhängig voneinander lineares oder verzweigtes $C_2$-$C_6$-Alkylen und t eine Zahl von 2 bis 6 sind. Sofern die Gruppe $C_yH_{2y}$ durch $-NR^5-$ unterbrochen ist, kann die Gruppe vorzugsweise der Formel

$$-(-R^8-NH-)_s-R^9-$$

entsprechen, worin $R^8$ lineares oder verzweigtes $C_2$-$C_6$-Alkylen, bevorzugt Ethylen, $R^9$ lineares oder verzweigtes $C_1$-$C_{10}$-, vorzugsweise $C_2$-$C_6$-Alkylen und s eine Zahl von 1 bis 3 sind. In Formel IV steht y besonders für eine Zahl von 2 bis 7.

Eine bevorzugte Untergruppe sind solche Amine der Formel IV, worin $R^5$ für H steht, y eine Zahl von 2 bis 7 und x eine Zahl von 1 bis 3 bedeuten.

Beispiele für Amine der Formel IV sind: Ethanolamin, 1-Amino-2-hydroxypropan, 1-Amino-3-hydroxypropan, 1-Amino-4-hydroxybutan, 1-Amino-5-hydroxypentan, 1-Amino-6-hydroxyhexan, Aminotrimethylolmethan, Aminodimethylolmethan, Aminomethyldimethylolmethan, Aminomethyltrimethylolmethan, Hydroxyethoxyethylamin, Hydroxypropoxyethylamin, N-(Hydroxyethyl)ethylendiamin, N-(Hydroxyethyl)-diethylentriamin,

$$H_2N(CH_2CH_2O-)_{2-6}H.$$

Primäre aliphatische Hydroxylgruppen enthaltende Amine sind Härter für Epoxidharze, wobei lineare Polymere erhalten werden, wenn Epoxidharze mit 2 Epoxidgruppen verwendet werden. Bei Mitverwendung anderer Härter werden vernetzte Polymere erhalten.

Bei Verwendung der monofunktionellen Anthrachinone der Formel I werden vorteilhaft Epoxidharze mit mindestens 3 Epoxidgruppen im Molekül verwendet, z.B. epoxidierte Novolake, um vernetzte Epoxidharze zu erhalten.

Die erfindungsgemässen Zusammensetzungen sind härtbar, wobei die gehärteten bzw. vernetzten Epoxidharze lichtempfindlich sind. Auf den belichteten Stellen der Oberfläche können durch stromlose Metallabscheidung dünne Schichten von Metallen wie z.B. Kupfer abgeschieden werden.

Die Härtung der Zusammensetzung erfolgt in bekannter Weise, wobei vor oder mit der Härtung eine Formgebung nach den üblichen Formgebungsverfahren möglich ist, z.B. die Herstellung von Beschichtungen auf einem Trägermaterial durch Spritzen, Streichen oder Rakeln, oder die Herstellung von Formteilen mittels Giesstechniken, oder die Herstellung von Verbundkörpern mittels Tränk- und Pressverfahren.

Das Anthrachinon der Formel I kann mit einem Epoxidharz zu Addukten vorreagiert werden, die dann mit dem Hydroxylgruppen enthaltenden Amin und gegebenenfalls einem Härter ausgehärtet werden können.

Es kann vorteilhaft sein, einen Härter für Epoxidharze mitzuverwenden, besonders einen Novolak-, Amin- oder Anhydridhärter. Zweckmässig wird das monofunktionelle Anthrachinon zusammen mit dem -OH enthaltenden Amin und dem Epoxid vorreagiert und darauf mit einem Härter vermischt und ausgehärtet.

Neben einer stufenweisen Härtung ist auch das Vermischen aller Komponenten und die darauffolgende Härtung möglich.

Das Vermischen der Komponenten erfolgt nach üblichen Verarbeitungsmethoden gegebenenfalls zusammen mit einem Lösungsmittel. Es können weitere für die Verarbeitung oder die Verbesserung der

Eigenschaften der gehärteten Epoxidharze übliche Zusätze zugegeben werden, z.B. Weichmacher, Farbstoffe, Pigmente, Füllstoffe, Formtrennungsmittel oder H-Donoren. Für die Abscheidung von Metallen kann ein Gehalt an Metallsalzen oder Metallkomplexen der Gruppen Ib oder VIII des periodischen Systems der Elemente vorteilhaft sein, z.B. in einer Menge von 0,01 bis 10 Gew.-%, bezogen auf die Zusammensetzung.

Die Härtung wird im allgemeinen bei Temperaturen von 20 bis 200°C, besonders 50 bis 150°C vorgenommen.

Die gehärteten Zusammensetzungen sind ein weiterer Gegenstand der Erfindung.

Die gehärteten Zusammensetzungen sind lichtempfindlich. Die belichteten Teile erscheinen dunkler als die unbelichteten Teile. Auf den belichteten Teilen können mit konventionellen Metallabscheidungsbädern (siehe z.B. US-PS 4 510 279), besonders solchen mit z.B. Nickel- oder Kupfersalzen, direkt Metalle abgeschieden werden. So können z.B. gedruckte Schaltungen hergestellt werden. Die belichteten Epoxidharze können auch für optische Speicherungen verwendet werden.

Ein weiterer Gegenstand der Erfindung ist die Verwendung einer gehärteten erfindungsgemässen Zusammensetzung zur Herstellung von metallischen Ueberzügen oder Abbildungen durch stromlose Metallabscheidung nach einer vollständigen oder teilweisen Belichtung der Oberfläche.

Die Belichtung wird bevorzugt mit UV-Licht vorgenommen. Es können beliebige Lichtquellen eingesetzt werden, wobei die Verwendung von UV-Lampen bevorzugt ist. Geeignete Lichtquellen sind z.B. Xenonlampen, Metallhalogenidlampen und besonders Quecksilberhoch-und -mitteldrucklampen.

Zur Herstellung der metallischen Ueberzüge oder Abbildungen kann man so vorgehen, dass man die erfindungsgemäss, sich gegebenenfalls als Schicht auf einem Trägermaterial befindliche erfindungsgemässe Zusammensetzung härtet, darauf flächenmässig oder durch eine Bildvorlage belichtet und danach mit einem Metallabscheidungsbad behandelt.

Die Mitverwendung eines Metallsalzes oder Metallkomplexes ist überflüssig. Die abgeschiedenen Metalle haften fest auf der Epoxidharzoberfläche; eine Vorbehandlung ist nicht notwendig. Ferner weisen die gehärteten Zusammensetzungen erhöhte Glasumwandlungstemperaturen auf.

Die nachfolgenden Beispiele erläutern die Erfindung näher.

## A) Herstellungsbeispiele

### Beispiel 1: Anthrachinon-2-carbonsäure-hydrazid

Eine Mischung von Anthrachinon-2-carbonsäure-ethylester (4,83 g), 25 ml Ethanol, 50 ml Dioxan und 7 ml Hydrazinhydrat wird unter Argon 48 Stunden gekocht. Man giesst auf Wasser, filtriert und trocknet bei 60°C im Vakuumofen. Kristallisation des Rohproduktes (4,05 g) aus Dimethylformamid/Chloroform gibt 3,01 g Produkt, Schmelzpunkt (Smp.) 250°C/Zersetzung.

Massenspektrum (indirekte Probenzuführung, 195°C): m/e = 266 ($M^+$, 20 %), 235 (100 %).

### Beispiel 2: (Anthrachino-2-yl)-essigsäure-hydrazid

14,7 g (Anthrachino-2-yl)-essigsäure-ethylester werden unter Erwärmen in 500 ml absolutem Ethanol gelöst. Nach Zugabe von 10 ml Hydrazinhydrat wird 20 Stunden unter Rückfluss gekocht. Es werden weitere 10 ml Hydra zinhydrat zugegeben und nochmals 20 Stunden gekocht. Man filtriert heiss und wäscht das Filtergut mit 50%-igem wässrigem Ethanol. Kristallisation des Rohproduktes (12,5 g) aus 300 ml Dimethylformamid gibt 11,2 g Produkt, Smp. über 300°C.

Analyse (Gew.-%):

| | | | | |
|---|---|---|---|---|
| Berechnet: | C 68,57 | H 4,32 | N 10,00 | O 17,13 %. |
| Gefunden: | C 68,9 | H 4,4 | N 9,8 | O 17,2 %. |

### Beispiel 3: 3-(Anthrachino-2'-yl)-propionsäurehydrazid

a) (Anthrachino-2-yl)-malonsäurediethylester

48,53 g (200 mMol) 2-Chloranthrachinon, 160,17 g (1 Mol) Malonsäurediethylester und 500 ml Dimethylsulfoxid (DMSO) werden auf 120°C erwärmt, bis alles gelöst ist. Dann wird auf 110°C gekühlt und portionenweise 165,85 g (1,2 Mol) Kaliumcarbonat zugegeben. Nach 1 Tag Rühren bei 110°C wird das Gemisch abgekühlt, mit HCl-Lösung versetzt und dreimal mit Toluol extrahiert. Die Extrakte werden dreimal mit Wasser gewaschen, über Natriumsulfat getrocknet und eingedampft. Durch Filtrieren mit Toluol über

Kieselgel und Umkristallisieren aus Methylenchlorid/Pentan werden 38,53 g (53 %) reines Produkt erhalten, Schmelzpunkt: 114-117°C.

Elementaranalyse (Gew.-%):

| | | | |
|---|---|---|---|
| Berechnet: | C 68,85 | H 4,95 | O 26,20 %. |
| Gefunden: | C 68,77 | H 5,14 | O 25,89 %. |

b) 2-(Anthrachino-2′-yl)-2-carboethoxy-bernsteinsäurediethylester

20,0 g (54,6 mMol) Verbindung 3a) werden mit 22,63 g (164 mMol) Kaliumcarbonat, 18,23 g (109,2 mMol) Bromessigsäureethylester und 200 ml DMSO während 1 Stunde bei 25°C gerührt. Das Gemisch wird mit Toluol und verdünnter HCl-Lösung versetzt. Durch zweimaliges Extrahieren der Wasserphase mit Toluol, Waschen der Toluolphasen mit Wasser, Trocknen über Natriumsulfat und Eindampfen erhält man einen kristallinen Rückstand, der nach Verrühren mit Diethylether, Abfiltrieren und Trocknen 19,77 g (80 %) reines Produkt ergibt, Schmelzpunkt: 80-84°C.

Elementaranalyse (Gew.-%):

| | | | |
|---|---|---|---|
| Berechnet: | C 66,36 | H 5,35 | O 28,29 %. |
| Gefunden: | C 66,65 | H 5,40 | O 28,54 %. |

c) Anthrachino-2-yl)-bernsteinsäure

16,35 g (292 mMol) Kaliumhydroxid werden in 400 ml absolutem Ethanol gelöst und 22,0 g (48,6 mMol) Verbindung 3b) zugegeben. Das Gemisch wird während 20 Minuten bei 25°C gerührt und dann während 30 Minuten zum Rückfluss erhitzt. Nach dem Abkühlen wird mit 2N wässriger HCl-Lösung versetzt und mit THF/Toluol (1:1) extrahiert. Die organischen Phasen werden über Natriumsulfat getrocknet und eingedampft. Durch Verrühren mit Diethylether, Abfiltrieren und Trocknen erhält man 15,51 g (98 %) reines Produkt, Schmelzpunkt: 245-250°C (Zersetzung)

Elementaranalyse (Gew.-%):

| | | | |
|---|---|---|---|
| Berechnet: | C 66,67 | H 3,73 | O 29,60 %. |
| Gefunden: | C 66,98 | H 4,02 | O 28,83 %. |

d) 3-(Anthrachino-2′-yl)-propionsäure

10 g Dicarbonsäure c) werden in 70 ml Chinolin unter Argon während 5 Stunden auf 140°C erwärmt. Man giesst auf 400 ml Eiswasser, säuert mit 2n Salzsäure an und filtriert. Das Filtergut wird mit Wasser gewaschen, getrocknet (7,9 g) und aus 300 ml Eisessig umkristallisiert. Ausbeute 5,3 g, Smp. 251°C.

Elementaranalyse (Gew.-%):

| | | | |
|---|---|---|---|
| Berechnet: | C 72,85 | H 4,32 | O 22,83 %. |
| Gefunden: | C 72,2 | H 4,3 | O 23,4 %. |

e) 3-(Anthrachino-2'-yl)-propionsäure-ethylester

5 g Säure 3d) werden in 80 ml Ethanol mit 3 ml konzentrierter Schwefelsäure 5 Stunden unter Rückfluss erhitzt. Man lässt erkalten, filtriert das kristallisierte Produkt ab, wäscht mit 50%-igem wässrigem Ethanol und reinigt den Ester (4,3 g) durch Kristallisation aus 100 ml Ethanol. Ausbeute 3,7 g, Smp. 101°C.

Elementaranalyse (Gew.-%):

| | | | |
|---|---|---|---|
| Berechnet: | C 74,02 | H 5,23 | O 20,76 %. |
| Gefunden: | C 73,9 | H 5,3 | O 20,8 %. |

f) 3-(Anthrachino-2'-yl)-propionsäure-hydrazid

9,2 g Ester e) werden in 200 ml Ethanol mit 6 ml Hydrazinhydrat während 72 Stunden unter Argon erhitzt. Man filtriert heiss und wäscht mit Ethanol und 50%-igem Ethanol-Wasser. Das Rohprodukt (4,6 g) wird aus 200 ml Dioxan umkristallisiert, Ausbeute 3,7 g, Smp. 229°C/Zersetzung.

Elementaranalyse (Gew.-%):

| | | | | |
|---|---|---|---|---|
| Berechnet: | C 69,38 | H 4,80 | N 9,52 | O 16,31 %. |
| Gefunden: | C 68,9 | H 4,9 | N 9,5 | O 16,6 %. |

Beispiel 4: 10-(Anthrachino-2'-yl)-decansäurehydrazid

a) 10-Phenyl-decansäure-ethylester

Eine Lösung von 26,15 g 9-Benzoyl-nonansäure in 260 ml Eisessig und 1 ml 0,1N Salzsäure wird bei 30-35°C mit 2,6 g Palladium (5 Gew.-%) auf Kohle als Katalysator hydriert (Normaldruck). Wenn kein Wasserstoff mehr aufgenommen wird, filtriert man und dampft das Filtrat bei reduziertem Druck ein. Der Rückstand wird in 200 ml Ethanol und 2 ml konzentrierter Schwefelsäure gelöst und die Lösung 22 Stunden unter Rückfluss erhitzt. Das Lösungsmittel wird abgedampft, der Rückstand mit Wasser versetzt und mit Ether extrahiert. Die organische Phase wird abgetrennt, mit Wasser, Natriumbicarbonat-Lösung (10 Gew.-%) und mit gesättigter Kochsalzlösung gewaschen, getrocknet und eingedampft. Destillation des Rückstandes am Hochvakuum (1 mbar, 137-141°C) gibt 20 g 10-Phenyl-decansäure-ethylester.

b) 2-[4'-(9''-Ethoxycarbonyl-nonyl)-benzoyl]-benzoesäure

Zu einer Lösung von 18 g 10-Phenyl-decansäure-ethylester in 100 ml 1,2-Dichloroethan werden bei 0-3°C 9,6 g Phthalsäureanhydrid und portionenweise innert 1 Stunde 26 g Aluminiumchlorid zugegeben. Nach 20 Stunden Rühren bei Raumtemperatur wird auf Eis/2N Salzsäure gegossen und mit Dichloromethan extrahiert. Waschen der organischen Phase mit gesättigter Kochsalzlösung, Trocknen und Eindampfen gibt 25,1 g 2-[4'-(9''-Ethoxycarbonyl-nonyl)-benzoyl]-benzoesäure als wachsartigen Festkörper, welcher ohne Reinigung weiterverwendet werden kann.

c) 10-(Anthrachino-2'-yl)-decansäure

Zu 120 ml auf 90°C vorgeheiztem 10%igem Oleum werden unter Argon 12,7 g 2-[4'-(9''-Ethoxycarbonyl-nonyl)-benzoyl]-benzoesäure gegeben und 1 Stunde bei 90°C gerührt. Die Mischung wird auf 400 ml Eiswasser gegossen und filtriert. Das Filtergut wird mit Wasser aufgeschlämmt und in Essigsäureethylester aufgenommen. Trocknen der organischen Phase, Eindampfen und Kristallisation des Rückstandes (7,2 g) auf Essigester gibt 4,1 g 10-(Anthrachino-2'-yl)-decansäure, Smp. 135-137°C, Massenspektrum (indirekte Probenzuführung, 210°C): m/e = 378 ($M^+$, 40 %), 222 (100 %).

d) 10-(Anthrachino-2'-yl)-decansäure-ethylester

Eine Lösung von 4 g 10-(Anthrachino-2'-yl)-decansäure in 80 ml Ethanol und 3 ml konzentrierter

Schwefelsäure wird 24 Stunden unter Rückfluss erhitzt. Man kühlt auf 0° C, filtriert und wäscht mit wässrigem Ethanol (30 %). Kristallisation des Filtergutes aus Methanol gibt 3,7 g Produkt, Smp. 81° C.

Elementaranalyse (Gew.-%):

| | | | |
|---|---|---|---|
| Berechnet: | C 76,82 | H 7,44 | O 15,47 %. |
| Gefunden: | C 76,7 | H 7,4 | O 15,8 %. |

e) 10-(Anthrachino-2′-yl)-decansäure-hydrazid

Eine Lösung von 20 g des Esters d) in 400 ml Ethanol wird nach Zugabe von 10 ml Hydrazinhydrat 24 Stunden unter Rückfluss erhitzt. Man gibt 15 ml Hydrazinhydrat zu und kocht weitere 24 Stunden. Man filtriert heiss, konzentriert das Filtrat durch Abdampfen des Lösungsmittels und filtriert nochmals. Kristallisation des Filtergutes (16,7 g) aus 350 ml Dioxan gibt 12,6 g Produkt, Smp. 190-191° C.

Elementaranalyse (Gew.-%):

| | | | | |
|---|---|---|---|---|
| Berechnet: | C 73,44 | H 7,19 | N 7,14 | O 12,23 %. |
| Gefunden: | C 73,7 | H 7,4 | N 7,1 | O 12,3 %. |

Beispiel 5: 10-(Anthrachino-2′-yl)-decansäure-N-I-methylhydrazid

Zu einer Suspension von 3,8 g 10-(Anthrachino-2′-yl)-decansäure in 40 ml Tetrahydrofuran werden bei -15° C unter Argon 1,26 g 4-Methylmorpholin und 1,5 g Chlorameisensäureisobutylester gegeben. Nach 5 Minuten Rühren bei -15° C wird die resuliertende Lösung mit 0,92 g N-Methylhydrazin in 10 ml Tetrahydrofuran versetzt. Es wird 30 Minuten bei Raumtemperatur gerührt, von den ausgefallenen Salzen abfiltriert, das Lösungsmittel abgedampft, in 50 ml Essigester aufgenommen und mit 20 ml wässriger Natriumbicarbonat-Lösung (5 Gew.-%) gewaschen. Der Rückstand der organischen Phase (3,3 g) wird an 500 g Kieselgel mit Chloroform/Essigester (7:1) chromatographiert. Kristallisation des Eluates (2,8 g) aus Toluol gibt 1,03 g Produkt, Smp. 66-67° C.

Elementaranalyse (Gew.-%):

| | | | | |
|---|---|---|---|---|
| Berechnet: | C 73,86 | H 7,44 | N 6,89 | O 11,81 %. |
| Gefunden: | C 74,4 | H 7,5 | N 6,4 | O 11,4 %. |

Beispiel 6: 3-[(Anthrachino-2′-yl-methoxy)-ethoxy]propionsäure-hydrazid

a) 2-(2′-Hydroxyethoxy)methyl-anthrachinon

Eine Suspension von 10,14 g 2-(Chlormethyl)-anthrachinon in 50 ml Ethylenglycol wird 14 Stunden bei 160° C gerührt. Man nimmt in Chloroform auf, wäscht mit Wasser und gesättigter Kochsalzlösung. Kristallisation des Rückstandes der organischen Phase (12 g) aus Toluol gibt 9,0 g 2-(2′-Hydroxyethoxy)methyl-anthrachinon, Smp. 129-130° C.

b) 3-[(Anthrachino-2′-yl-methoxy)-ethoxy]propionsäure-methylester

Eine Mischung von 264 mg Verbindung a), 370 mg Kaliumcarbonat und 1 ml Acrylsäuremethylester in 5 ml Dimethylsulfoxid wird 2 Tage bei Raumtemperatur unter Argon gerührt. Man verdünnt mit Wasser, extrahiert mit Chloroform, trocknet die organische Phase und destilliert die Lösungsmittel ab. Chromatographie des Rückstandes an Kieselgel mit Dichloromethan/Ethylacetat (10:1) als Eluens gibt 137 mg Produkt. Massenspektrum (indirekte Probenzuführung, 190° C): m/e = 368 ($M^+$, 30 %), 87 (100 %).

c) 3-[(Anthrachino-2′-yl-methoxy)-ethoxy]propionsäure-hydrazid

Eine Lösung von 43 mg des Methylester b) und 0,1 ml Hydrazinhydrat in 3 ml Ethanol wird 20 Stunden unter Rückfluss erhitzt. Man verdünnt mit Dichlormethan, wäscht mit Wasser und gesättigter Kochsalzlösung, trocknet die organische Phase und dampft das Lösungsmittel ab. Chromatographie an Kieselgel mit Dichlormethan/ Ethanol (12:1) als Eluens gibt 10 mg Hydrazid. Massenspektrum (indirekte Probenzuführung, 220°C): m/e = 368 ($M^+$, 10%), 221 (100 %).

B) Anwendungsbeispiele

Beispiele 7-10:

11,91 g Bisphenol-A-diglycidylether und 4,95 g 10-(Anthrachino-2′-yl)-decylcarbonsäure-hydrazid werden unter Inertgas und mechanischem Rühren auf 180°C erhitzt. Man erhält eine viskose, klare Schmelze von gelber Farbe. Das Epoxyäquivalent der Schmelze beträgt 2,18 mol Epoxid/kg (bestimmt nach der Methode von R.R. Jay, Analytical Chemistry 36 (1964) 667).

Man löst in 40 ml N-Methylpyrrolidon auf und versetzt mit 1,13 g 1,3 Propanolamin und 1,23 g eines Phenol-Formaldehyd-Novolaks mit einem Hydroxyäquivalent von 123,15 g/Mol OH. Die Lösung wird mittels Drahtrakeln als Film auf Aluminium oder Polyesterträger gegossen und im Umluftofen für 12 Stunden bei 80°C getrocknet, und dann bei 140°C für 4 Stunden ausgehärtet.

Bei Beispiel 8 wird analog verfahren. Beispiele 9 und 10 werden in Lösung durchgeführt, wie nachfolgend für Beispiel 9 beschrieben.

8,41 g 3-(Anthrachino-2′-yl)-propionsäurehydrazid und 23,82 g Bisphenol-A-diglycidylether werden in 50 ml N-Methylpyrrolidon unter Inertgas und mechanischem Rühren auf 120°C erhitzt. Man erhält eine viskose, klare Lösung von gelber Farbe. Man lässt abkühlen und fügt 2,25 g 1,3-Propanolamin sowie 1,23 g eines Kresol-Formaldehyd-Novolaks mit einem Hydroxyäquivalent von 123,15 g/Mol OH zu und lässt bei Raumtemperatur rühren. Die Lösung wird mittels Drahtrakeln als Film auf Aluminium oder Polyesterträger gegossen und im Umluftofen für 12 Stunden bei 80°C getrocknet, und dann bei 140°C für 4 Stunden ausgehärtet.

Tabelle 1 zeigt Zusammensetzung und Eigenschaften der gehärteten Harze.

Die Effizienz der Photoreduktion wird wie folgt bestimmt. Ein Film auf einem Polyesterträger mit der optischen Dichte (O.D.) = 1 bei 324 nm wird mit einer Hg-Hochdrucklampe mit 40 $mW/cm^{-2}$ bestrahlt und in regelmässigen Abständen das UV/VIS-Spektrum aufgenommen. Die Bande bei 324 nm nimmt ab, die Bande bei 386 nm nimmt zu. Das Verhältnis beider Banden nach 2 Minuten Belichtungszeit wird als Effizienz genommen.

Photometallisierung

Filme auf einem Polyesterträger werden auf einem thermostatisierbaren Vakuumheiztisch bei 50°C durch ein Negativ mit einer Hg-Hochdrucklampe mit 40 $mW/cm^{-2}$ Intensität belichtet. Man erhält ein dunkles, negatives Abbild der Vorlage, das in einem Abscheidungsbad der Zusammensetzung (siehe US-PS 4,510,276):

$CuSO_4$ x 5H2O 0,0665 Mol/l
HCOH 0,0467 Mol/l
Quadrol 0,0599 Mol/l
NaOH pH12,6
NaCN 25 mg/l
2-Mercaptobenzthiazol 10 mg/l

bei 45°C zu einem metallischen Kupferbild verstärkt wird.

<u>Tabelle 1:</u>

| Bsp. Nr. | Anthrachinon (AQ) | Menge (mMol) | | | | Tg [°C] | $\frac{O.D._{386\ nm}}{O.D._{324\ nm}}$ |
|---|---|---|---|---|---|---|---|
| | 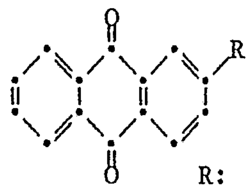 R: | AQ | 1-Amino-propan-3-ol | Bisphenol-A-diglycidyl-ether | Novolak (Aequival.) | | |
| 7 | $-(CH_2)_9-\overset{O}{\overset{\|}{C}}NHNH_2$ | 3 | 7 | 3 | 2 | 76 | 0,51 |
| 8 | $-(CH_2)_2-\overset{O}{\overset{\|}{C}}NHNH_2$ | 3 | 7 | 3 | 2 | 107 | 0,55 |
| 9 | $-CH_2\overset{O}{\overset{\|}{C}}NHNH_2$ | 3 | 7 | 3 | 2 | 102,8 | 0,62 |
| 10 | $-\overset{O}{\overset{\|}{C}}NHNH_2$ | 1 | 1 | 3 | - | 101 | 0,71 |

Beispiele 11-14:

Gemäss Beispiel 9 werden Epoxidharze aus 1 Miliäquivalent Novolak, 3 mMol 2-Propanolamin, einer Mischung aus 1 mMol mit Bisphenol-A vorverlängertem Bisphenol-A-diglycidylether (Epoxyäquivalent 901 g/Mol) und 6 mMol 5,5'-Dimethyl-hydantoin-N,N'-diglycidylether, und 3 mMol Carbonsäurehydrazid der Formel

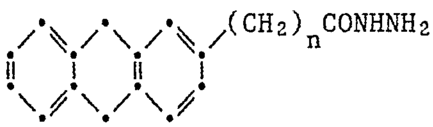

hergestellt. Ansonsten wird wie in den Beispielen 7-10 verfahren. Die Ergebnisse sind in Tabelle 2 zusammengefasst.

Tabelle 2:

| Beispiel | n | Tg (°C) | $\frac{O.D._{386\ nm}}{O.D._{324\ nm}}$ |
|---|---|---|---|
| 11 | 0 | 84 | 0,40 |
| 12 | 1 | 76 | 0,52 |
| 13 | 2 | 75 | 0,61 |
| 14 | 9 | 72 | 0,64 |

**Patentansprüche**

1. Anthrachinone der Formel I

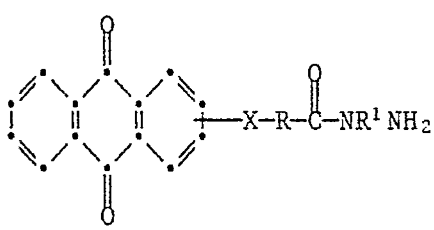

(I),

worin
X für die Gruppe $-CR^2R^3$-steht, $R^2$ H, -CN oder $C_1$-$C_5$-Alkyl und $R^3$ H oder -CN sind, $R^1$ H oder $C_1$-$C_5$-Alkyl darstellt, und R eine direkte Bindung oder lineares oder verzweigtes $C_1$-$C_{18}$-Alkylen ist, das alleine oder zusammen mit der $-CR^2R^3$-Gruppe durch ein oder mehrere -O- unterbrochen sein kann, wenn $R^2$ und/oder $R^3$ nicht -CN sind.

2. Anthrachinone der Formel I gemäss Anspruch 1 worin die Gruppe

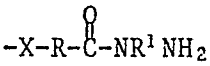

in 2-Stellung gebunden ist.

3. Anthrachinone der Formel I gemäss Anspruch 1, worin X für die Gruppe $-CR^2R^3-$ steht, in der $R^2$ H oder $C_1$-$C_5$-Alkyl und $R^3$ H bedeuten.

4. Anthrachinone der Formel I gemäss Anspruch 1, worin $R^2$ als Alkyl Methyl oder Ethyl ist.

5. Anthrachinone der Formel I gemäss Anspruch 3, worin $R^2$ und $R^3$ H sind.

6. Anthrachinone der Formel I gemäss Anspruch 1, worin R lineares Alkylen ist.

7. Verfahren zur Herstellung von Anthrachinonen der Formel I gemäss Anspruch 1 und entsprechende Verbindungen, worin -XR- eine direkte Bindung darstellt, dadurch gekennzeichnet, dass man einen Anthrachinoncarbonsäureester der Formel II

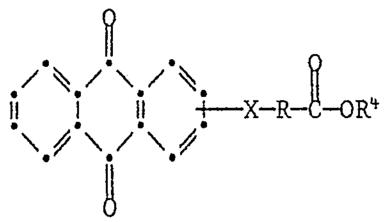 (II),

worin X und R die in Anspruch 1 angegebenen Bedeutungen haben oder die Gruppe -XR- eine direkte Bindung darstellt und $R^4$ O- für den Rest eines Alkohols stehen, in Gegenwart eines Alkohols mit einem Hydrazin der Formel III

$HNR^1NH_2$ (III),

worin $R^1$ die in Anspruch 1 angegebene Bedeutung hat, oder mit dessen Hydrat oder Salzen umsetzt.

8. Verfahren gemäss Anspruch 7, dadurch gekennzeichnet, dass der Alkohol ein $C_1$-$C_4$-Alkanol ist.

9. Verfahren gemäss Anspruch 7, dadurch gekennzeichnet, dass $R^4$ $C_1$-$C_6$-Alkyl ist.

10. Härtbare Zusammensetzung, enthaltend

a) mindestens ein Epoxidharz mit durchschnittlich mehr als einer Epoxidgruppe im Molekül,

b) gegebenenfalls einen Härter für das Epoxidharz,

c) mindestens ein Anthrachinon der Formel I gemäss Anspruch 1, wobei die Gruppe -X-R- zusätzlich die Bedeutung einer direkten Bindung haben kann, und

d) mindestens ein primäres oder sekundäres aliphatisches Amin, das im aliphatischen Rest mindestens eine Hydroxylgruppe enthält.

11. Zusammensetzung gemäss Anspruch 10, worin

c) das Anthrachinon der Formel I in einer Menge von 0,1-1 Mol/kg Epoxidharz, und

d) das Amin in einer Menge von 0,1-1,2 Mol/kg Epoxidharz enthalten sind.

12. Zusammensetzung gemäss Anspruch 10, worin als Epoxidharz glycidylierte Novolake, Hydantoine, Aminophenole, Bisphenole oder aromatische Diamine enthalten sind.

13. Zusammensetzung gemäss Anspruch 12, worin das Epoxidharz ein epoxidierter Kresolnovolak, Bisphenol-A-diglycidylether, mit Bisphenol-A vorverlängerter Bisphenol-A-diglycidylether, 5,5-Dimethylhydantoin-N,N'-bisglycid, 1,3-Bis(N,N'-diglycidyl-5,5-dimethylhydanto-4-yl)-2-glycidoxypropan, p-Aminophenoltriglycid, Diaminodiphenylmethan oder Mischungen hiervon ist.

14. Zusammensetzung gemäss Anspruch 10, worin es sich bei dem Härter b) um einem Novolak, ein Polyamin oder ein Polycarbonsäureanhydrid handelt.

15. Zusammensetzung gemäss Anspruch 10, worin das Amin der Komponente d) der Formel IV

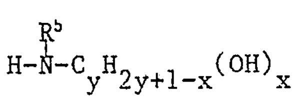 (IV)

entspricht, worin $R^5$ H, lineares oder verzweigtes $C_1$-$C_{18}$-Alkyl, $C_3$-$C_7$-Cycloalkyl, $C_6$-$C_{10}$-Aryl, $C_7$-$C_{18}$-Alkaryl, $C_7$-$C_{12}$-Aralkyl, $C_8$-$C_{18}$-Alkaralkyl oder die Gruppe $-C_yH_{2y+1-x}(OH)_x$ bedeutet, x für eine Zahl von 1 bis 3 und y für eine Zahl von 2 bis 12 stehen, wobei die Gruppe $C_yH_{2y}$ durch ein oder mehrere -O- oder $-NR^5-$ unterbrochen sein kann.

16. Zusammensetzung gemäss Anspruch 10, worin in Formel IV $R^5$ für H steht, y eine Zahl von 2 bis 7 und x eine Zahl von 1 bis 3 bedeutet.

17. Gehärtete Zusammensetzung aus

a) mindestens einem Epoxidharz mit durchschnittlich mehr als einer Epoxidgruppe im Molekül,
b) gegebenenfalls einem Härter für Epoxidharz,
c) mindestens einem Anthrachinon der Formel I gemäss Anspruch 1, wobei die Gruppe -XR- zusätzlich die Bedeutung einer direkten Bindung haben kann, und
d) mindestens ein primäres oder sekundäres aliphatisches Amin, das im aliphatischen Rest mindestens eine Hydroxylgruppe enthält.

18. Verwendung einer gehärteten Zusammensetzung gemäss Anspruch 17 zur Herstellung von metallischen Ueberzügen oder Abbildungen durch stromlose Metallabscheidung nach einer vollständigen oder teilweisen Belichtung der Oberfläche.

19. Verfahren zur Herstellung von metallischen Ueberzügen oder Abbildungen auf einem Epoxidharz, bei dem man eine Zusammensetzung gemäss Anspruch 10 härtet, darauf flächenmässig oder unter einer Bildvorlage belichtet und danach mit einem Metallabscheidungsbad behandelt.